(19) Europäisches Patentamt
European Patent Office
Office européen des brevets

(11) EP 1 308 728 A2

(12) **EUROPÄISCHE PATENTANMELDUNG**

(43) Veröffentlichungstag:
**07.05.2003 Patentblatt 2003/19**

(51) Int Cl.$^7$: **G01N 33/533**, G01N 33/58, G01N 33/68, C12Q 1/68

(21) Anmeldenummer: **02024602.1**

(22) Anmeldetag: **04.11.2002**

(84) Benannte Vertragsstaaten:
**AT BE BG CH CY CZ DE DK EE ES FI FR GB GR IE IT LI LU MC NL PT SE SK TR**
Benannte Erstreckungsstaaten:
**AL LT LV MK RO SI**

(30) Priorität: **05.11.2001 DE 10153818**

(71) Anmelder: **Chromeon GmbH**
**93053 Regensburg (DE)**

(72) Erfinder:
- **Yarmoluk, Sergey M.**
  **04108 Kiew (UA)**
- **Kostenko, Olexandr M.**
  **03143 Kiew (UA)**
- **Tolmachev, Olexandr Iwanowitsch**
  **02150 Kiew (UA)**
- **Wolfbeis, Otto S.**
  **93051 Regensburg (DE)**

(74) Vertreter: **Dey, Michael, Dr. et al**
**Weickmann & Weickmann,**
**Patentanwälte,**
**Postfach 86 08 20**
**81635 München (DE)**

(54) **Verfahren und Verbindungen zur Fluoreszenzmarkierung von Biomolekülen und Polymerpartikeln**

(57) Die Erfindung betrifft somit ein Verfahren, mit dessen Hilfe es möglich ist, Aminogruppen-tragende bioorganische Moleküle wie z.B. Aminosäuren, Proteine, Pharmaka, Antikörper, Aminogruppen-modifizierte Nucleotide, aber auch Aminogruppen-tragende Polymere und Polymerpartikel, über eine chemische (kovalente) Bindung mit einem Fluoreszenzmarker zu versehen. Das Verfahren beruht auf der Umsetzung eines an einem Fluorophor *F* befindlichen Pyryliumsalzes mit der Aminogruppe eines Biomoleküls oder Partikels im Sinne der angeführten Reaktionsgleichung. Das Verfahren ist selektiv, einfach durchführbar und ergibt hohe Markierungsgrade. Die spektralen Eigenschaften der Konjugate unterscheiden sich deutlich von jenen der Ausgangsverbindungen, und ihre Fluoreszenz-Quantenausbeuten sind oft deutlich erhöht.

EP 1 308 728 A2

## Beschreibung

Die Erfindung betrifft ein Verfahren zur Fluoreszenzmarkierung von Aminogruppen-tragenden Substanzen, hierfür geeignete Fluoreszenzmarker sowie deren Verwendung in fluoreszenz-analytischen oder diagnostischen Bestimmungsverfahren.

Die Fluoreszenzmarkierung von Biomolekülen spielt eine wichtige Rolle in der Bioanalytik und biologischen Forschung. Man unterscheidet zwischen Fluorophoren, die nicht kovalent an Biomoleküle wie z.B. Proteine oder DNA binden, und solchen, die kovalent an Biomoleküle, aber auch an Partikel gebunden werden können.

Das allgemeine Reaktionsschema bei allen bekannten Verfahren kann wie folgt dargestellt werden: An einem Fluorophor *F* befindet sich eine Gruppe *X*, die mit einer an einem Biomolekül oder Partikel befindlichen zweiten Gruppe (z. B. *HY*) eine chemische Reaktion (oder auch nur eine starke Wechselwirkung wie zum Beispiel zwischen Biotin und Avidin) eingeht. Bei der Bildung chemischer (kovalenter) Bindungen wird dabei entweder eine Gruppe vom Typ XH abgespalten, typischerweise nach folgender Reaktionsgleichung:

F-X + HY-Biomolekül = = > *F*-Biomolekül + XH

Die Konjugation kann aber auch im Sinn einer Additionsreaktion nach folgender Gleichung erfolgen:

F-X + HY-Biomolekül = = > *F*-XH-Y-Biomolekül

Auf diese Weise gelingt es, einen Fluorophor F in ein Molekül einzuführen und dieses somit allen fluoreszenz-analytischen Verfahren zugänglich zu machen. Typische Beispiele für die Gruppen *X* und *Y* bzw. für (a) Substitutions-, (b) Additions- und (c) Bindungsreaktionen sind in der folgenden Tabelle 1 angegeben:

| X (am Fluorophor) | Y (am Biomolekül oder Partikel) | Reaktionstyp (Abgangsgruppe bzw. neue Gruppe) |
|---|---|---|
| $-SO_2Cl$ | $R'-NH_2$ | Substitution (-HCl) |
| $-CO-CH_2-I$ | R'-SH | Substitution (-HI) |
| $-CO-CH_2-Br$ | R'-COOH | Substitution (-HBr) |
| -CO-O-NHS*) | $R'-NH_2$ | Substitution (-N-Hydroxysuccinimid) |
| -NCS | $R'-NH_2$ | Addition<br>(-NH-CS-NH-R') |
| -NCO | R'-OH (Alkohol) | Addition<br>(-NH-CO-OR') |
| -Maleinimid | R'-SH | Addition |
| -Biotin | R'-Avidin | Affinitätsbindung<br>(nicht kovalent) |
| -Avidin | R'-Biotin | Affinitätsbindung<br>(nicht kovalent) |

*)NHS = N-Hydroxysuccinimid

Die reaktiven Gruppen (X) sind allerdings oft schwer durch chemische Synthese einzufügen und leiden auch unter dem Nachteil, dass sie nicht lagerstabil sind. Schon Spuren von Wasser können derartige Gruppen langsam (durch Hydrolyse) zersetzen, worauf sie unreaktiv werden. Ähnliches gilt für die Biotinylierung.

Eine Aufgabe der vorliegenden Erfindung war es deshalb ein Verfahren zur Fluoreszenzmarkierung bereitzustellen, welches die genannten Nachteile nicht aufweist. Insbesondere sollten Hydrolyse- und lagerbeständige Fluoreszenzmarker bereitgestellt werden.

Diese Aufgabe wird erfindungsgemäß gelöst durch ein Verfahren zur Fluoreszenzmarkierung von Aminogruppen-tragenden Substanzen, welches dadurch gekennzeichnet ist, dass man einen Fluoreszenzfarbstoff, der zumindest eine reaktive Pyryliumgruppe der folgenden Strukturen A, B oder C enthält

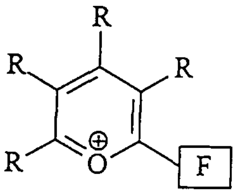 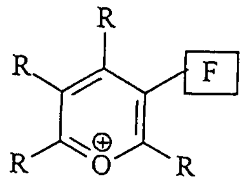 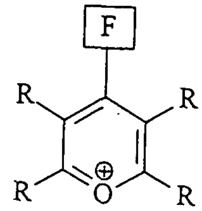

A B C

worin
F für einen Fluorophor steht,
R für einen Rest steht, der die Fluoreszenz des Fluorophors nicht löscht und die Reaktion der Pyryliumgruppe mit Aminen nicht behindert,
mit der primären Aminogruppe der Aminogruppen-tragenden Substanz umsetzt, dass sich gemäß der Reaktion

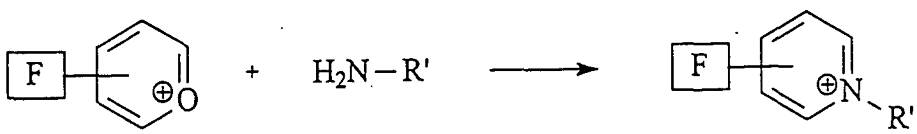

D

ein Pyridiniumsalz der Struktur D bildet
in dem F für einen Fluorophor, und
R' für einen (bio)organischen Rest steht.

Das hier beschriebene neue Verfahren beschreibt Fluorophore einer Reaktivgruppe (X), die eine der folgenden chemischen Strukturen A, B oder C aufweist:

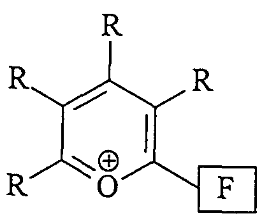 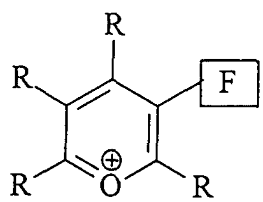 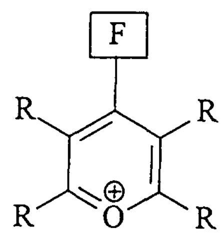

A B C

Hier steht *F* für einen beliebigen Fluorophor, und *R* für einen beliebigen, überwiegend organischen Substituenten, der die Fluoreszenz des Systems nicht löscht bzw. die Reaktion des Pyryliumsalzes mit Aminen nicht behindert. Diese Art von reaktiver Gruppe wird hierin als Pyrylium-Gruppe bezeichnet.

Es wurde nun gefunden, dass sich derartige Farbstoffe dazu verwenden lassen, Aminogruppen-tragende Spezies fluoreszent zu markieren. Unter relativ milden Bedingungen und sowohl in wässrigen wie in organischen Lösungsmitteln läuft mit primären Aminen ($R'-NH_2$) folgende Reaktion ab:

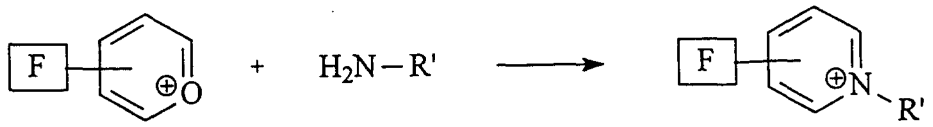

wobei F wieder für einen beliebigen Fluorophor steht und R' für einen (bio)organischen, insbesondere einen aliphatischen oder aromatischen Rest mit z.B. 1 bis 30, insbesondere 1 bis 20 C-Atomen, der auch beliebig substituiert sein kann. Auf diese Weise hat man aus einem nicht markierten Biomolekül mit einer Aminogruppe (R'-$NH_2$) ein mit *F* fluoreszenz-markiertes Biomolekül erhalten. Mit sekundären Aminen (R'-NH-R') bzw. tertiären Aminen ($NR'_3$) erfolgt naturgemäß keine Markierung.

Das erfindungsgemäße Verfahren kann im Allgemeinen zur Markierung von Substanzen eingesetzt werden, welche mindestens eine primäre Aminogruppe enthalten.

Als primäre Amine kommen bevorzugt in Frage aliphatische und aromatische Amine, Aminosäuren und aminomodifizierte Biomoleküle und Pharmaka, aber auch synthetische Materialien und Polymere bzw. Polymerpartikel - mit freien Aminogruppen. Die Polymerpartikel haben vorzugsweise einen Durchmesser zwischen 0,1 und 20 µm, mehr bevorzugt zwischen 1 und 10 µm.

Die Gruppe F kann erfindungsgemäß ein beliebiger Chlorophor sein, also ein Rest, welcher fluoreszierende Eigenschaften hat. Die Reste R an der Pyryliumgruppe sind vorzugsweise Wasserstoff oder Kohlenwasserstoffreste mit 1 bis 30 C-Atomen, vorzugsweise 1 bis 10 C-Atomen. Beispiele für besonders bevorzugte Reste R sind Wasserstoff, Methyl, tert-Butyl und Phenyl. Besonders bevorzugt sind Pyryliumverbindungen, in denen die Gruppe F in Paraposition angeordnet ist, also Verbindungen der Struktur C. Weiterhin ist bevorzugt, dass der Rest R in den Position 2 und 6 (ortho) von Wasserstoff verschieden ist.

Die erfindungsgemäßen Fluoreszenzmarker eignen sich insbesondere zur Markierung von Aminogruppen-tragenden Substanzen für Fluoreszenzanalytische oder diagnostische Bestimmungsverfahren. Durch kovalentes Binden des Analyten, also einer Aminogruppen-tragenden Substanz an die erfindungsgemäßen Fluoreszenzmarker kann ein einfacher optischer Nachweis des Analyten erfolgen. Die erfindungsgemäßen Fluoreszenzmarker zeichnen sich insbesondere dadurch aus, dass die Pyryliumgruppe eine kovalente chemische Reaktion mit primären Aminogruppen eingehen kann, was zur kovalenten Fluoreszenzmarkierung von Substanzen, welche eine Aminogruppe enthalten, führt. Das hier beschriebene Verfahren betrifft somit Marker, die kovalent an Biomoleküle (und Partikel) mit darin vorhandenen Aminogruppen binden können.

Die Erfindung betrifft somit insbesondere ein Verfahren, mit dessen Hilfe es möglich ist, Aminogruppen-tragende bioorganische Moleküle wie z.B. Aminosäuren, Proteine, Pharmaka, Antikörper, Aminogruppen modifizierte Nucleotide, aber auch Aminogruppen-tragende Polymere und Polymerpartikel, über eine chemische (kovalente) Bindung mit einem Fluoreszenzmarker zu versehen. Das Verfahren beruht auf der Umsetzung eines an einem Fluorophor *F* befindlichen Pyryliumsalzes mit der Aminogruppe eines Biomoleküls oder Partikels im Sinne der angeführten Reaktionsgleichung. Das Verfahren ist selektiv, einfach durchführbar und ergibt hohe Markierungsgrade. Die spektralen Eigenschaften der Konjugate unterscheiden sich deutlich von jenen der Ausgangsverbindungen, und ihre Fluoreszenz-Quantenausbeuten sind oft deutlich erhöht.

Die Reaktion wird anhand folgender typischer Beispiele erläutert.

*Beispiel 1. Synthese des Markerfarbstoffes Cyan 39: (1-Methyl-2-[4-(2,6-dimethyl-1,4-dihydropyryliden)methyl]-benzo-1,3-thiazolium-perchlorat*

Man löst 5,5 g 1,2-Dimethyl-benzo-1,3-thiazolium-methosulfonate und 2,48 g 2,6-Dimethyl-4-pyron mit 1 Tropfen Perchlorsäure in 10 mL Acetanhydrid und erhitzt 4 h unter Rückfluss. Danach wird die Reaktionsmischung mit 20 mL Ethanol verdünnt und mit 2 mL einer gesättigten wässrigen Natriumperchlorat-Lösung versetzt. Nach 2 - 3 h saugt man den entstandenen gelben Niederschlag ab und kristallisiert ihn aus Ethanol um.

*Beispiel 2. Synthese des Markerfarbstoffes Cyan 58: 2,6-Dimethyl-4-[3-(1-methyl-2,3-dihydro-1,3-benzthiazol-2-ylyden)-1-propenyl]-pyryliumperchlorate*

Eine Mischung aus 150 mg 2,4,6-Trimethylpyrylium-tetrafluoroborat und 218 mg 2-[2-(Acetanilinovinyl-1 )]-3-methylbenzthiazolium-lodid wird mit 50 mg Triethylamin in 5 mL 100 %igem Ethanol 1 h zum Sieden erhitzt. Danach werden 500 µL einer gesättigten wässrigen Lösung von Natriumperchlorat zugegeben. Nach dem Abkühlen wird der entstandene blau-violette Niederschlag abfiltriert und durch Chromatographie auf Aluminiumoxid (Laufmittel Dichlormethan) gereinigt. Die blau-violette Fraktion wird isoliert und nach Verdunsten des Laufmittels durch Kristalisation aus Ethanol rein erhalten.

Die Tabellen 2-4 zeigen weitere chemische Strukturen von erfindungsgemäßen Farbstoffen, die Absorptionsmaxima der freien Pyryliumsalze (linke Spalten) bzw. deren Konjugate an die Aminoäure Glycin (rechte Spalten). Die längstwelligen Absorptionsmaxima der Konjugate sind üblicherweise kürzer als jene der nicht konjugierten Pyrylium-Farbstoffe. Die Spektren werden von der Art des Amines bzw. der Aminosäure praktisch nicht beeinflusst. Es ist aus der Tabelle aber ersichtlich, dass durch Variation des Substituenten *F* am Pyrylium-Salz fast beliebige viele Farben eingestellt werden können.

Tabelle 2. Chemische Strukturen von Pyryliumsalzen (links) und deren Umsetzungsprodukten mit Glycin (R = CH2-COOH) (rechts)

| Nr. | chem. Struktur | $\lambda_{max}$, nm | Nr. | chem. Struktur | $\lambda_{max}$, nm |
|---|---|---|---|---|---|
| 1 | 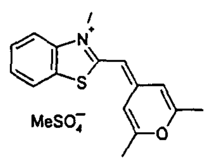 | 432, 460 | 2 | 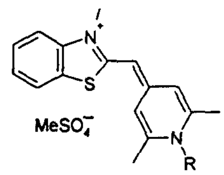 | 440 |
| 3 | 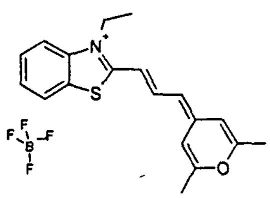 | 536 572 | 4 | 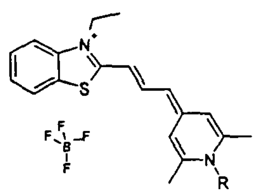 | 540 |
| 5 | 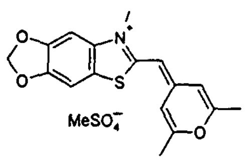 | 461 480 | 6 | 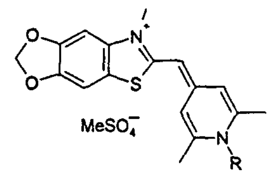 | 460 |
| 7 | 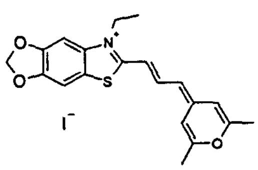 | 558 592 | 8 | 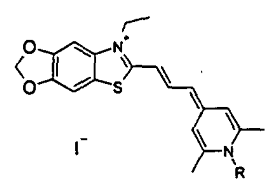 | 567 |

Tabelle 3. Weitere Beispiele von Pyryliumsalzen (links) und deren Umsetzungsprodukten mit Glycin (R = CH2-COOH) (rechts)

| Nr. | chem. Struktur | $\lambda_{max}$, nm | Nr. | chem. Struktur | $\lambda_{max}$, nm |
|---|---|---|---|---|---|
| 9 | 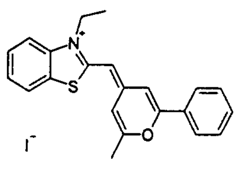 | 460<br>483 | 10 | 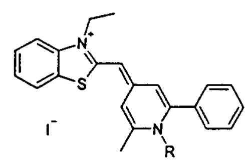 | 450 |
| 11 | 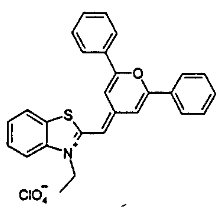 | 476,<br>502 | 12 | 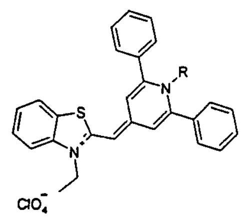 | 462 |
| 13 | 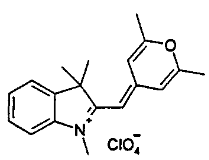 | 453 | 14 | 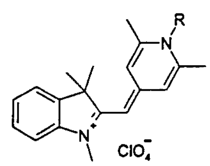 | 432 |
| 15 | 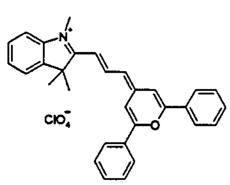 | 589,<br>628 | 16 | 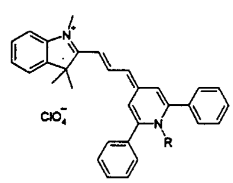 | 550 |
| 17 | 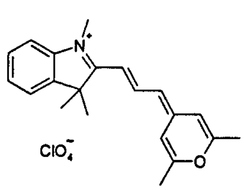 | 554,<br>575 | 18 | 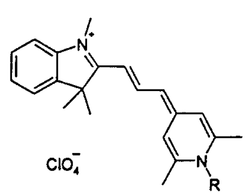 | 509 |
| 19 | 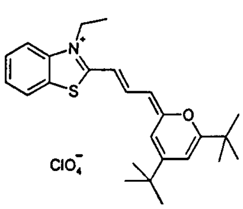 | 587,<br>626 | 20 | 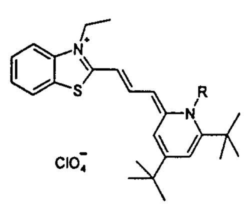 | 547 |

Die Absorptionsmaxima der Pyrylium-Farbstoffe (linke Spalten in den Tabellen 2-4) besitzen üblicherweise 2 Absorptionsbanden, nämlich eine intensivere im langwelligeren Bereich und eine schwächere im etwas kurzwelligeren Bereich. Die Konjugate mit dem Amin besitzen hingegen üblicherweise eine einzige starke Bande, die etwa im Bereich der kurzwelligeren Bande des Pyrylium-Salzes liegt. Das Absorptionsspektrum der Verbindung nach Beispiel 2 ist in **Figur 1** dargestellt. Die durchgezogene Linie des blauvioletten Markers CCyan 39 ist das typischen 2-Bandenspektrum eines Pyryliumsalzes vor einer Konjugation an eine Aminogruppe. Die gestrichelte Linie ist das typische 1-Bandenspektrum des Farbstoffes nach der Konjugation an die Aminosäure Glycin (Kurve CCyan 40).

*Beispiel 3. Konjugation an Aminosäuren*

Die oben beschriebenen Marker wurden zur Fluoreszenz-Markierung von Aminosäuren eingesetzt. Als Lösungsmittel dienten TEAA-Puffer (Triethylammonium-Acetat) und Dimethylsulfoxid. Die jeweilige Aminosäure wurde in alkalischem Puffer (>10) in einer Konzentration von 0.1 Mol/L gelöst. Der Farbstoff Cyan 39 wurde in 2 mL DMSO gelöst (0.01 Mol/L) und langsam in die auf 50 °C erwärmte Lösung der Aminosäure getropft. Die Reaktion wurde über die Abnahme der Lichtabsorption bei 470 nm verfolgt. Cyan-39 hat ein Absorptionsmaximum bei 470 nm, das Konjugat hat sein Maximum bei 434 nm. Cyan-58 kann in vollkommen analoger Weise eingesetzt werden. Die Konjugate können durch präparative Chromatographie (wie oben beschrieben) gereinigt werden.

Die gelb bzw. rot markierten Aminosäuren bilden sich besonders rasch, wenn die Lösung bei pH-Werten über 10 durchgeführt wird. Die folgende Tabelle 4 gibt eine Übersicht über die Reaktionszeiten als Funktion der eingestellten pH-Werte. Daraus ist ersichtlich, dass die Umsetzung mit der Aminosäure Lysin (die in Proteinen vorzugsweise markiert wird) bei pH-Werten zwischen 11 und 12 innerhalb von 15 - 20 min vollständig ist.

*Tabelle 4. Dauer der Reaktion (in Minuten) bis zur mindestens 90%-igen Umsetzung von Aminosäuren mit dem Marker Cyan-39 in wässrigem TEAA-Puffer bei verschiedenen pH-Werten*

| pH-Wert | ∈-ACS | Lys | Ser | Arg | Gly | Ala | Val | Trp | His | Asp |
|---|---|---|---|---|---|---|---|---|---|---|
| 12,0 | 11 | 13 | 30 | 38 | 8 | 47 | 51 | 56 | 70 | 66 |
| 11,4 | 18 | 20 | 35 | 51 | 16 | 66 | 78 | 106 | 113 | 109 |
| 11,2 | 33 | 26 | 62 | 166 | 22 | 85 | 104 | 130 | 145 | 185 |

*Beispiel 4. Markierung eines amino-modifizierten Oligomers*

Das amino-modifizierte 15-Oligomere 3'-TAA-TGG-CCT-GAG-ATAT-$(CH_2)_6$-$NH_2$ wurde mit dem Reaktivfarbstoff Cyan-58 auf folgende Weise umgesetzt: Das Oligomer (0.2 mg) wurde in 5 mL Acetonitril gelöst und auf 50 °C erwärmt. Danach wurde eine Lösung von 0.1 mg des Farbstoffes Cyan-58 langsam zugetropft. Nach 1 h wurde das Acetonitril abgedunstet und der Rückstand einer Polyacrylamid-Elektrophorese unterworfen. Oligomer und restlicher (überschüssiger) Farbstoff lassen sich leicht trennen. Der freie Marker ist blauviolett und absorbiert maximal bei 572 nm. Das Konjugat an das Oligomer ist rotviolett und absorbiert maximal bei 540 nm.

*Beispiel 5. Markierung von Glas-Mikropartikeln*

Poröse Glaskügelchen (1.0 g; Porengröße 70 nm; mit Aminopropylgruppen an der Oberfläche (40 - 100 μMol pro Gramm Kügelchen; bezogen von Sigma, Produkt-Nr. G-5019) wurde in Pufferlösung von pH 10 suspendiert. Dazu wurde langsam und unter raschem Rühren bei 50 °C eine Lösung von 3 mg des blauen Farbstoffes Cyan-58 (siehe oben) in DMSO zugetropft. Nach einer Stunde wurden die violett angefärbten Glasspartikel abgesaugt, mit reichlichen Mengen destilliertem Wasser, 1 %-iger Essigsäure und wieder Wasser gewaschen, bis kein Farbstoff mehr im Waschwasser zu finden war. Danach wurden die Partikel getrocknet und im trockenen Zustand gelagert. Die violett gefärbten Partikel weisen eine starke orangerote Fluoreszenz auf.

*Beispiel 6. Markierung von Polystyrol-Mikropartikeln*

Zur Demonstration einer Fluoreszenzmarkierung von Polystyrol-Partikeln wurden Teilchen mit einem durchschnittlichen Durchmesser von 160 - 200 μm eingesetzt, die an den Oberflächen Aminomethylgruppen trugen (Produkt 81558, von Fluka, Buchs; Schweiz). 1.0 g dieser Partikel wurden in Ethanol suspendiert, mit überschüssigem Farbstoff

Cyan 39 (10 mg; siehe Beispiel 1) versetzt und 2 h unter Rückfluss erhitzt. Die so markierten Partikel sind orangerot gefärbt und weisen eine grüne Fluoreszenz auf.

*Beispiel 7. Markierung eines Proteins*

Zu 100 µL einer Lösung von Lysozyme (Sigma) in Wasser (2 mg/mL, ≈ $1{,}5 \cdot 10^{-4}$ M) wurden 100 µL einer 0,1 M Lösung von Triethylammoniumacetat-Puffer (von pH 12.1) gegeben. Danach fügte man 100 µL einer Lösung ($1{,}5 \cdot 10^{-3}$ M) des Farbstoffes Cyan-39 in DMSO zu. Die Reaktionsmischung wurde für 2 h bei 50 °C gehalten. Danach wurde die Lösung auf eine Sephadex-Säule aufgebracht (Sephadex G-25, Säule: 1 cm x 30 cm, 0,01 M TEAA Puffer, pH 12,1) und der orangerote freie Farbstoff auf diese Weise vom gelben Lysozym-Konjugat getrennt. Die gelbe Proteinfraktion läuft viel schneller als der Farbstoff.

**Patentansprüche**

1. Verfahren zur Fluoreszenzmarkierung von Aminogruppen-tragenden Substanzen, **dadurch gekennzeichnet, dass** man einen Fluoreszenzfarbstoff, der zumindest eine reaktive Pyryliumgruppe der folgenden Strukturen A, B oder C enthält

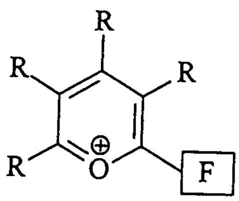

A

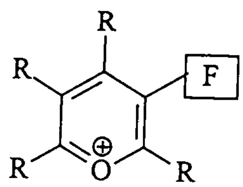

B

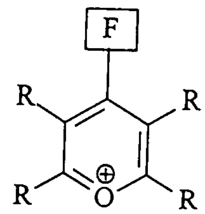

C

worin
F für einen Fluorophor steht,
R für einen Rest steht, der die Fluoreszenz des Fluorophors nicht löscht und die Reaktion der Pyryliumgruppe mit Aminen nicht behindert,
mit der primären Aminogruppe der Aminogruppen-tragenden Substanz umsetzt, dass sich gemäß der Reaktion

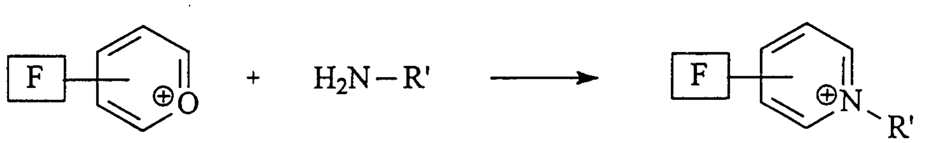

D

ein Pyridiniumsalz der Struktur D bildet
in dem F für einen Fluorophor, und
R' für einen (bio)organischen Rest steht.

2. Verfahren nach Anspruch 1, **dadurch gekennzeichnet, dass** die Aminogruppen-tragende Substanz ausgewählt wid aus bioorganischen Molekülen, Pharmaka oder Aminogruppen-tragenden Polymeren bzw. Polymerpartikeln mit einem Durchmesser zwischen 0.1 und 20 µm.

3. Verfahren nach Anspruch 1 oder 2, **dadurch gekennzeichnet, dass** $R'-NH_2$ ein Biomolekül, insbesondere eine Aminosäure, ein organisches Amin, ein Pharmakon, ein Protein oder ein aminomodifiziertes Nucleotid oder dessen Oligomer ist.

**4.** Verfahren nach einem der Ansprüche 1 oder 2, **dadurch gekennzeichnet, dass** die Aminogruppen-tragende Substanz ein Polymer oder ein Polymerpartikel mit einem Durchmesser von 0.01 - 10 µm ist.

**5.** Fluoreszenzmarker zur Markierung von Substanzen mit primärer Aminogruppe, **dadurch gekennzeichnet, dass** er die allgemeine Struktur A, B oder C aufweist, welche ein Fluorophor und eine Pyryliumgruppe umfasst,

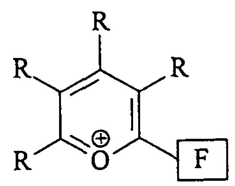

A

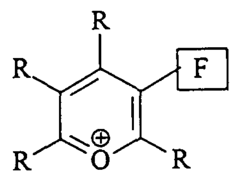

B

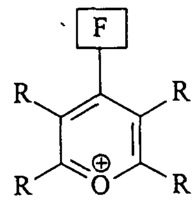

C

wobei
R für einen Rest steht, der die Fluoreszenz des Fluorphores nicht löscht und die Reaktion des Fluoreszenzmarkers mit primären Aminen nicht verhindert.

**6.** Fluoreszenz nach Anspruch 5 zur Markierung von organischen Verbindungen, insbesondere für bioorganische Moleküle und Pharmaka, sowie für Polymere bzw. Polymerpartikel mit einem Durchmesser von typischerweise zwischen 0.1 und 10 µm.

**7.** Fluoreszenz-markierte Substanz umfassend einen Fluoreszenzmarker nach Anspruch 5 oder 6 oder erhältlich durch ein Verfahren nach einem der Ansprüche 1 bis 4.

**8.** Verwendung einer fluoreszenz-markierten Substanz nach Anspruch 8 fluoreszenz-analytischen oder diagnostischen Bestimmungsverfahren.

**9.** Verwendung nach Anspruch 8, **dadurch gekennzeichnet, dass** das Bestimmungsverfahren auf der Messung der Intensität, der Abklingzeit, der Löschung, der Effizienz des Energietransfers oder der Polarisation der Fluoreszenz beruht.

**10.** Verwendung nach Anspruch 8 oder 9, **dadurch gekennzeichnet, dass** das Bestimmungsverfahren auf der Verwendung einer Mikrotiterplatte und eines Mikrotiterplattenlesers, eines Fließcytometers oder eines anderweilig automatisierten Laborverfahrens beruht.

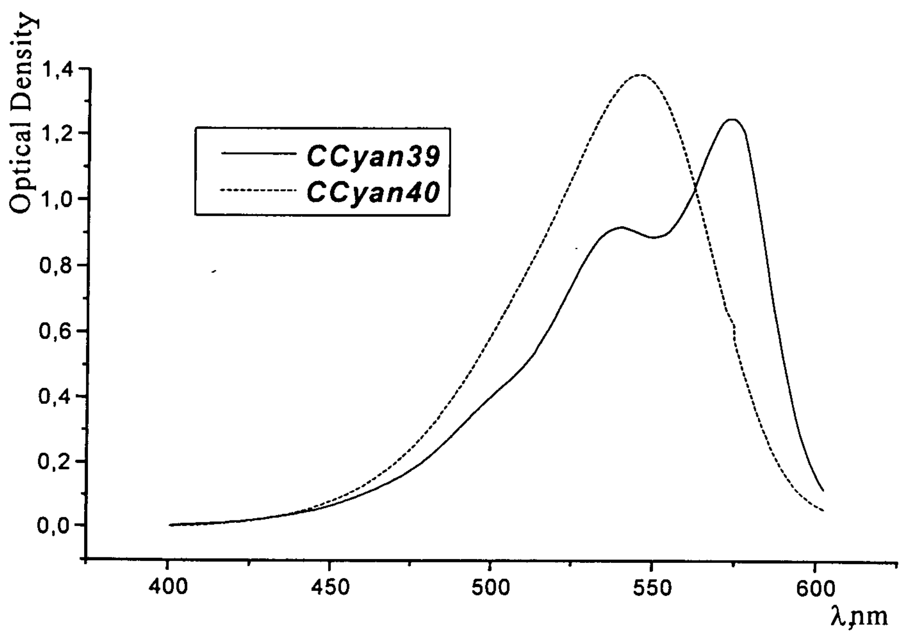
Optical Density
1,4
1,2
1,0
0,8
0,6
0,4
0,2
0,0
CCyan39
CCyan40
400
450
500
550
600
λ,nm

Figur 1